# EUROPEAN PATENT APPLICATION

(11) **EP 2 783 705 A1**
(43) Date of publication of application: **01.10.2014**
(21) Application number: 12850802.5
(22) Date of filing: 20.08.2012
(51) Int. Cl.: A61L 27/00, A61F 2/28, A61F 2/30, A61K 6/00, C22C 14/00

(54) **BIOMEDICAL METALLIC POROUS PLATE**

(30) Priority: 24.11.2011 JP 2011256673
(71) Applicant: Kyocera Medical Corporation, Osaka-shi, Osaka 532-0003 (JP)
(72) Inventor: ISHIMIZU, Keita, Osaka-shi Osaka 532-0003 (JP); YAMASHITA, Mitsuyoshi, Osaka-shi Osaka 532-0003 (JP)
(74) Representative: Müller-Boré & Partner Patentanwälte PartG mbB
(86) International application number: PCT/JP2012/070993
(87) International publication number: WO 2013/077046

(57) **Abstract**

The present invention aims to obtain a surface modification member which is bonded successfully to an implant material in a method for bonding a surface modification member and an implant material by using a slurry containing metal powders. The present invention is a biocompatible metallic porous plate to be bonded to the surface of in vivo implant material, wherein the area ratio A of open holes (a) present on the surface of the side facing the implant material is less than the area ratio B of open holes (b) present on the surface of the side facing a living body; and the open holes (a) communicate through to the living body side. The biocompatible metallic porous plate is preferably produced by layered manufacturing.

## Description

### TECHNICAL FIELD

The present invention relates to a metallic porous plate to be bonded to the surface of in vivo implant material.

### BACKGROUND ART

Implant materials such as an artificial bone, an artificial joint, and an artificial dental root to be embedded and used in a living body are required to be excellent in compatibility with biotissues. One of means for attaining compatibility with biotissues is a method for bonding a surface modification member made of a porous body or the like to the surface of the implant material. For the method, in addition to the necessity of improvement of in vivo compatibility by the surface modification member, it is necessary that the surface modification member and the surface of the implant material are bonded to each other with sufficient strength.

For example, in Patent Document 1, for the purpose of bonding a metallic porous body having excellent bonding properties to biotissues to the surface of a medical device main body with high bonding strength, the metallic porous body is produced by multi-layering of metallic porous thin sheets and the metallic porous body is bonded to the surface of a medical device. In Patent Document 1, the metallic porous body and the medical device are bonded by diffusion bonding. However, if there is a gap in the bonding surface, concern for diffusion bonding is a decrease in bonding strength and an unevenness in bonding strength in the gap. Normally, pressurization to the bonding surface is required during thermal treatment for the purpose of improving the bonding strength, but since thermal treatment is carried out at about 1000°C in the diffusion bonding, a material of a stone weight for the pressurization is limited or an exclusive tool is required in the case of pressurization to a complicated shape, and thus there has been another problem in terms of labors and costs.

### PRIOR ART DOCUMENT

### PATENT DOCUMENT

Patent Document 1: JP-A 2007-151805

### SUMMARY OF THE INVENTION

### PROBLEMS TO BE SOLVED BY THE INVENTION

Accordingly, the inventors of the present invention focus on a method for bonding a surface modification member and an implant material by using a slurry containing metal powders, and aim to obtain a surface modification member which is bonded successfully to an implant material in the method.

### MEANS FOR SOLVING THE PROBLEMS

The present invention is a biocompatible metallic porous plate to be bonded to the surface of in vivo implant material, wherein the area ratio A of open holes (a) present on the surface of the side facing the implant material is less than the area ratio B of open holes (b) present on the surface of the side facing a living body; and the open holes (a) communicate through to the living body side. The biocompatible metallic porous plate is preferably produced by layered manufacturing.

For the biocompatible metallic porous plate, it is preferable that (i) the area ratio of the open holes (a) present on the surface of the side facing the implant material is 0.4 to 30%, a distance between neighboring open holes (a) in the surface is 0.5 to 7.0 mm, and the open hole (a) has a diameter not exceeding 600 µm, or that (ii) the open hole (a) has a thickness not exceeding 0.5 mm.

The present invention includes an in vivo implant material, wherein the above-mentioned biocompatible metallic porous plate is bonded to at least a portion of the surface of the implant material, and the biocompatible metallic porous plate is preferably bonded by using a slurry containing metal powders.

### EFFECTS OF THE INVENTION

According to the present invention, since the open holes (a) present on the surface of the side facing the implant material communicate through to the living body side, a binder component in a slurry for bonding the implant material and the metallic porous plate to each other is scattered in the form of a gas to the living body side through the open holes (a) at the time of heat treatment, and thus the formation of voids in the bonding surface of the implant material and the metallic porous plate is suppressed. Owing to this void suppression effect and the fact that the area ratio of the open holes (a) is less than the area ratio of open holes (b) present on the surface of the side facing the living body, the bonding strength of the implant material and the metallic porous plate is retained.

### BRIEF DESCRIPTION OF THE DRAWINGS

Fig. 1 is an image of a metallic porous plate of the present invention produced in Examples described below.
Fig. 2 is an observation photograph of the bonding surface of a metallic porous plate and a substrate in Examples described below.

### MODE FOR CARRYING OUT THE INVENTION

According to the investigation performed by the inventors of the present invention, it has been found that no sufficient bonding strength can be obtained in the case of bonding a metallic porous body having a porosity of 40 to 97%, which is disclosed in Patent Document 1, to an implant material by using a slurry containing metal powders. The reason for this is supposedly attributed to the fact that the bonding surface area is insufficient since the porosity of the porous body is high.

Therefore, the inventors of the present invention have tried to make the side facing the implant material in the metallic porous body be a dense surface without holes in order to increase the bonding surface area of the implant material and the metallic porous body. However, in this case, it has been found that if the implant material and the metallic porous body are bonded by applying slurry between them and thereafter heat treating, void parts are formed in the interface of them and sufficient bonding strength is not obtained. It is supposedly attributed to the fact that since there is no hole in the bonding surface and there is thus no escape route for gas even when the binder component in the slurry becomes a gas by the heat treatment, the gas is expanded and widened the bonding surface to be remained therein as voids.

According to the investigations, the inventors of the present invention have considered that sufficient bonding strength is obtained if a metallic porous plate is made to have a configuration of being capable of scattering the binder component vaporized by the heat treatment from the bonding surface while retaining the sufficient bonding surface area to the implant material. And this consideration has led to completion of the present invention. That is, in the metallic porous plate of the present invention, the area ratio A of open holes (a) present on the surface of the side facing the implant material is less than the area ratio B of open holes (b) present on the surface of the side facing a living body (A<B), and the open holes (a) communicate through to the living body side.

The open holes (b) are formed in terms of retention of compatibility with a living body, and their area ratio is usually set to be wide to a certain extent. However, if the area ratio of the open holes (a) present on the surface of the side facing the implant material is made to be equal to the area ratio of the open holes (b), the bonding surface area to the implant material is not retained. Therefore, in the present invention, the area ratio A of the open holes (a) is made less than the area ratio B of the open holes (b). In the present invention, the open holes (a) mean all of open holes present on the surface of the side facing the implant material, and the area ratio of the open holes (a) means the total area ratio of the open holes (a) to the surface of the side facing the implant material. Similarly, the open holes (b) mean all of open holes present on the surface of the side facing the living body, and the area ratio of the open holes (b) means the total area ratio of the open holes (b) to the surface of the side facing the living body.

Further, in the present invention, owing to the communication of the open holes (a) through to the living body side of the metallic porous plate, the binder component in the slurry is scattered to the living body side through the open holes (a) when the binder component is vaporized, so that the formation of voids on the bonding surface of the metallic porous plate and the implant material is suppressed. In the present invention, the fact that the open holes (a) communicate through to the living body side means not only the case where the open holes (a) communicate through to the living body side immediately above but also the case where the open holes (a) communicate through to the living body side through any holes other than the holes (a).

Further, the metallic porous plate has the open holes (a) on the side facing the implant material, and therefore, it is easy to be deformed on its surface as compared with the case where the side facing the implant material is made to be a dense plane without holes, and an effect of fitting the metallic porous plate easily in the implant material is also expected.

In order to more effectively exhibit the effect of the present invention, it is preferable to adjust the area ratio, hole diameter, or thickness of the open holes (a), or the distance between neighboring open holes (a).

The area ratio of the open holes (a) is preferably adjusted to 0.4 to 30%. The binder component in the slurry is efficiently scattered to the living body side by adjusting the area ratio of the open holes (a) to 0.4% or more. A sufficient bonding surface area to the implant material is retained and the bonding strength is improved by adjusting the area ratio of the open holes (a) to 30% or less. The lower limit of the area ratio of the open holes (a) is preferably 0.5% and more preferably 1 % (particularly 1.5%). The upper limit of the area ratio of the open holes (a) is preferably 27% and more preferably 25% (particularly 20%).

If the diameter of the open hole (a) is too wide, the slurry leaks to the open holes on the living body side (that is, the open holes other than the open holes (a) on the metallic porous plate) through the open holes (a) to lose the biocompatible function of the open holes on the living body side. Such leakage of the slurry is avoided by adjusting the viscosity of the slurry in accordance with the diameter of the open hole (a), but it is preferable to adjust the diameter of the open hole (a) not to exceed 600 µm. The adjustment in such a manner suppresses clogging of the open holes on the living body side with the slurry and thus excellent biocompatibility is exhibited. The diameter of the open hole (a) is more preferably not to exceed 500 µm, and even more preferably not to exceed 400 µm. From the viewpoint of prevention of leakage of the slurry, the hole diameter is more preferably as small as possible, and a preferable lower limit is usually, but is not particularly limited to, about 100 µm. The shape of the open hole (a) is not particularly limited. In the case where the open hole (a) has a circular shape, the diameter of the open hole (a) means a circle diameter, and in the case where the open hole (a) has a shape other than a circular shape, the diameter of the open hole (a) means an equivalent circle diameter (the value converted into a diameter of circle with the same surface area).

The open holes (a) are preferable to be present evenly on the side facing the implant material, and it is preferable to adjust the distance between neighboring open holes (a) to 0.5 to 7.0 mm. The binder component in the slurry is efficiently scattered to the living body side by the adjustment as described above. The lower limit of the distance between the neighboring open holes (a) is more preferably 1.0 mm, furthermore preferably 1.3 mm, and particularly preferably 1.5 mm, and the upper limit thereof is more preferably 6.5 mm, and furthermore preferably 6.0 mm. The distance between the open holes (a) means the distance between the centers of the open holes (a).

The open hole (a) has a thickness (that is, the open hole (a) is extended in parallel to the thickness direction) and the thickness is preferable to be as thin as possible and preferably not exceeding 0.5 mm from the viewpoint of retention of sufficient holes in the living body side in order to provide the biocompatibility. The thickness of the open hole (a) is more preferably not to exceed 0.4 mm and even more preferably not to exceed 0.3 mm. The lower limit of the thickness of the open hole (a) is usually, but is not particularly limited to, about 0.1 mm.

In particular, it is preferable to satisfy all of the conditions of the area ratio and diameter of the open holes (a) and the distance between neighboring open holes (a). The binder component in the slurry is scattered to the living body side through the open holes (a) by the satisfying the above conditions, and the formation of voids in the bonding surface of the metallic porous plate and the implant material is suppressed, and at the same time, the bonding surface area is sufficiently retained, so that the bonding strength is improved. Further, since the open holes for retaining the biocompatibility are not clogged with the slurry, excellent biocompatibility is also exhibited.

The area ratio of the open holes (b) is not particularly limited, and may be set properly in consideration of the compatibility with the living body, but it is usually about 50 to 85%. The porosity of holes in the living body side (that is, holes other than the holes (a)) is not also particularly limited, and may be set properly in consideration of the compatibility with the living body, but it is usually about 50 to 85%.

A metal for the metallic porous plate means both of a pure metal and an alloy. Metals to be used as the metal may be, for example, pure Ti, Ti alloys (e.g., Ti-6Al-4V, Ti-6Al-2Nb-1Ta, etc.) and Co alloys (e.g., Co-Cr alloy), because of having good biocompatibility. The thickness of the metallic porous plate is not particularly limited, but for example, about 0.7 to 5.0 mm and preferably 1.2 to 2.0 mm.

The metallic porous plate can be produced by, for example, layered manufacturing. The layered manufacturing is a method for producing a desired compact by spreading material powders in a layer form; and melting and thereafter solidifying the material powders by irradiating the powders with electromagnetic radiation rays such as laser beams or corpuscular radiation rays such as electron beams according to three-dimensional image digital data.

The material powders to be used for layered manufacturing can be prepared by an atomization method (water atomization method or gas atomization method), a rotary electrode method, a ball mill method, etc. It is preferable that powders prepared by the method are sieved if necessary to have an average particle diameter of usually about 20 to 150 µm (preferably about 30 to 60 µm).

The conditions for layered manufacturing may be set properly depending on the types of material powders to be used and the shape of the metallic porous plate (open hole diameter, porosity, distance between open holes, etc.).For example, the radiation diameter of radiation rays (e.g., circle diameter) may be adjusted to about 50 to 200 µm; the distance between the radiation ray source and the material powders may be adjusted to about 40 to 80 cm; and the layer thickness (thickness of one layer of a powder) may be adjusted to about 20 to 200 µm. The atmosphere at the time of layered manufacturing is not particularly limited, and it is preferable to carry out the layered manufacturing in a vacuum atmosphere. Further, the layered manufacturing may be carried out in an inert gas atmosphere such as argon gas or nitrogen gas.

The metallic porous plate is used while being bonded to at least a portion of the surface of in vivo implant material. In the present invention, a slurry containing metal powders is used in the bonding method. The gap between the implant material and the metallic porous plate is filled by using the slurry, and the deviation of the bonding strength can be made small as compared with that of a diffusion bonding method using no slurry. To be more specific, the bonding using a slurry can be carried out by applying a slurry containing metal powders to at least a portion of the surface of in vivo implant material; thereafter, attaching the metallic porous plate; and carrying out heat treatment. Consequently, the in vivo implant material in the present invention to which the metallic porous plate is bonded has a sintered metal powder layer which is formed in the interface between the metallic porous plate and the implant material, and the sintered metal powder layer is present at least a portion of the open holes (a) in the metallic porous plate.

Examples of the method for applying the slurry include brush coating, roller coating, spray coating, dip coating, etc.

The heat treatment temperature and heat treatment time may be set properly in consideration of the types of the metal powders in the slurry, the degree of sintering, sufficient vaporization of the binder component in the slurry, etc. The heat treatment temperature is, for example, about 800 to 1100°C (preferably about 900 to 1000°C) and the heat treatment time is about 1 to 5 hours (preferably about 2 to 4 hours).

The metal powder in the slurry may be a powder of either a metal or an alloy, and for example, pure Ti, Ti alloys (e.g., Ti-6Al-4V, Ti-6Al-2Nb-1 Ta, etc.), and Co alloys (e.g., Co-Cr alloy) can be used. The size of the metal powder in the slurry is not particularly limited, but for example, metal powders with an average particle diameter of about 20 to 150 µm can be used. Examples of the binder component in the slurry include saccharides such as starch and agarose, alcohols such as polyvinyl alcohol (PVA), etc. The content ratio of the metal powders in the slurry is, for example, about 75 to 95% by mass, and the content ratio of the binder component in the slurry is, for example, about 1% by mass.

This application claims the benefit of priority based on Japanese Patent Application No. 2011-256673 filed on November 24, 2011. The entire contents of the specification of Japanese Patent Application No. 2011-256673 filed on November 24, 2011 are incorporated herein by reference.

### EXAMPLES

Hereinafter, the present invention will be described more specifically with reference to Examples. The present invention is not limited to Examples described below, and can be naturally carried out by proper modifications within a range which is suitable to the gist described above and below. All of these are included in the technical scope of the present invention.

### Example 1

Pure Ti powders were prepared by an atomization method (average particle diameter was 30 µm). According to three-dimensional image digital data, the powders were irradiated with laser to produce a metallic porous plate. In detail, the metallic porous plate is composed of a 0.3 mm-thick plate-like member (50 mm × 50 mm × thickness 0.3 mm) having holes (a) which penetrate in the thickness direction and satisfying the conditions shown in Table 1 (open hole diameter, area ratio, and distance between open holes) and a 1.2mm-thick porous body formed thereon which has an area ratio of open holes of 65% in every surfaces vertical to the thickness direction (that is, the area ratio of open holes present on the surface of the side facing the living body was also 65%). The porous body part has a porosity of 65%. The layered manufacturing conditions were such that the laser beam radiation diameter was 100 µm and the layer thickness was 30 µm.

Subsequently, a slurry was prepared by mixing the pure Ti powders having an average particle diameter of 30 µm and an aqueous PVA solution (the pure Ti powder content was 87% by mass and the PVA content was 1% by mass in the slurry) and the slurry in an amount sufficient to cover the entire surface of the Ti alloy plate with the slurry was applied approximately to the center part of a Ti-6AI-4V alloy plate having the same size as that of the metal porous plate. Next, the metallic porous plate was stuck to the surface of the Ti alloy plate coated with the slurry in such a manner that the surface having the open holes (a) faced the Ti alloy plate, and after the slurry protruded out of the outer circumference was removed, the resulting product was dried at 100°C for 0.5 hours and subsequently heated at 950°C for 3 hours to obtain samples each simulating an implant material to which the metallic porous plate was bonded (Samples No. 1 to 10). For comparison, a sample No. 11 was prepared in the same manner as that for the samples No. 1 to No. 10, except that a plate-like member of Ti-6AI-4V having no hole was used in place of the plate-like member having the open holes (a).

The applied state of the slurry and the bonding state in the interface of the Ti alloy plate (substrate of the implant material) and the metallic porous plate were evaluated by the following methods.

### (1) Observation of leakage of slurry to surface of metallic porous plate

After the drying step in the sample preparation process described above, the leakage of the slurry to the surface (living body side) of the metallic porous plate was observed with naked eyes and evaluated according to the following criteria. Those marked with ⊚, ○, and Δ were regarded as passing.
⊚: No leakage is observed.
○: Leakage is observed but extremely a little.
Δ: Leakage is observed partially (in some points).
×: Leakage is observed in nearly the entire surface.

### (2) Observation of bonding surface after drying

After the drying step in the sample preparation process described above, the Ti alloy plate (substrate) and the metallic porous plate were separated from each other, and the bonding surface was observed with naked eyes and evaluated according to the following criteria. Those marked with ⊚, ○, and Δ were regarded as passing.
⊚. The Ti powders are densely present in the entire bonding surface of the substrate and the porous plate, and the surface of the substrate or porous body is not seen.
○: There are an extremely few portions where the Ti powders are present sparsely or no Ti powder is present in the bonding surface of the substrate and the porous plate.
Δ: There are some portions where the Ti powders are present sparsely or no Ti powder is present in the bonding surface of the substrate and the porous plate.
×: There are a wide range of portions where no Ti powder is present in the bonding surface of the substrate and the porous plate.

The results are shown in Table 1.

**[Table 1]**

| sample No | open hole diameter (µm) | area ratio of open hole part (%) | minimum value of distance between open holes (mm) | maximum value of distance between open holes (mm) | leakage of slurry | bonding surface state |
|---|---|---|---|---|---|---|
| 1 | 600 | 19.1 | 1.6 | 2.2 | Δ | ⊚ |
| 2 | 600 | 6.6 | 2.2 | 3.1 | Δ | ⊚ |
| 3 | 600 | 4.9 | 3.1 | 4.3 | Δ | ⊚ |
| 4 | 600 | 2.3 | 4.0 | 5.6 | Δ | ○ |
| 5 | 600 | 1.4 | 5.1 | 7.0 | Δ | Δ |
| 6 | 350 | 15.1 | 0.8 | 1.1 | ⊚ | ⊚ |
| 7 | 350 | 5.5 | 1.6 | 2.2 | ⊚ | ⊚ |
| 8 | 350 | 1.9 | 2.2 | 3.1 | ⊚ | ○ |
| 9 | 350 | 0.7 | 4.0 | 5.6 | ⊚ | Δ |
| 10 | 350 | 0.4 | 5.1 | 7.0 | ⊚ | Δ |
| 11 | plate without open hole | | | | ⊚ | × |

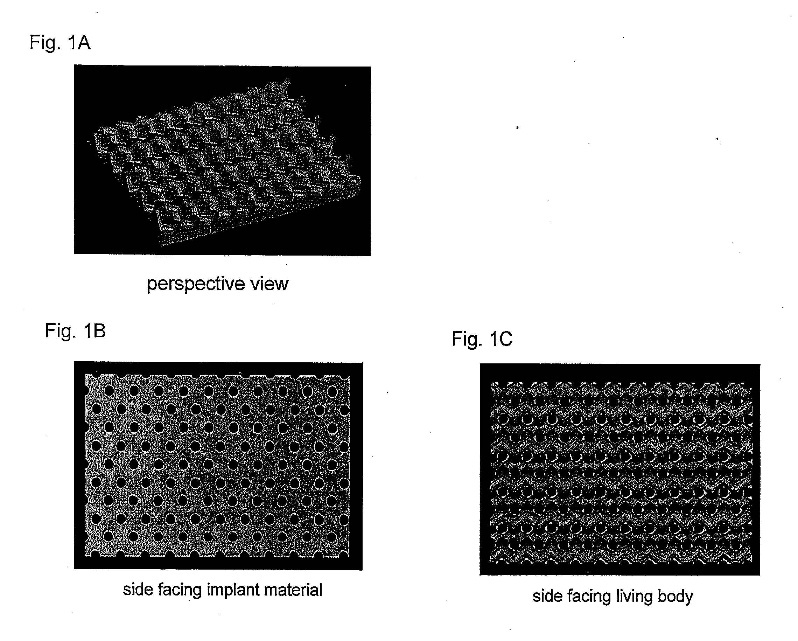

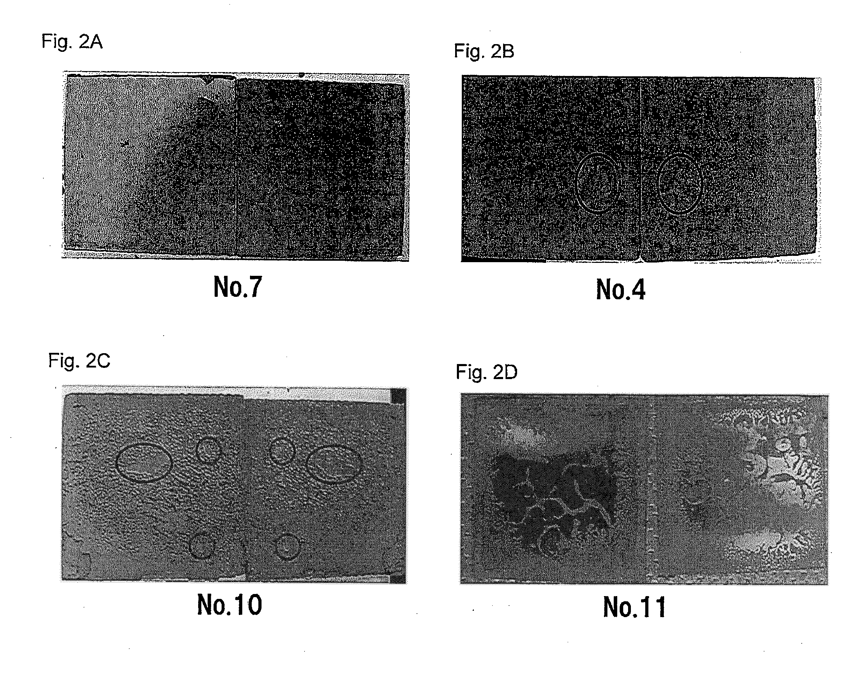

According to Table 1, it is understood that each of the samples No. 1 to No. 10 satisfying the requirements of the present invention has a good slurry leakage state and a good bonding surface state. Fig. 1 shows the image of a portion of the metallic porous plate in the sample No. 7 among the above-mentioned samples, and Figs.2A to 2D show the photographs of the bonding surfaces of the samples No. 4, 7, 10 and 11 after drying. In the sample No. 7 shown in Fig. 2A, a portion of the slurry adheres to the substrate side at the time of peeling the bonding surface, but the Ti powders are densely present in the entire surface, and the surfaces of the metallic porous plate and the substrate are not seen. In the sample No. 4 shown in Fig. 2B, there are observed portions where no Ti powders are present in the position marked with the circle and there are also a very few portions where the Ti powders are sparsely present. In the sample No. 10 shown in Fig. 2C, there are voids in some positions marked with the circle and there are also some portions where the Ti powders are sparsely present. In the sample No. 11 shown in Fig. 2D, there are portions where no Ti powder is present in a wide range of the center part of the bonding surface and the surfaces of the substrate and the metallic porous plate are exposed.

### Example 2

The sample No. 7 in Example 1 was subjected to a tensile test in accordance with ASTM (American Society for Testing and Materials) F-1147. For comparison, a sample No. 12 was prepared in the same manner as that for the sample No. 7, except that no plate-like member having open holes (a) was used (that is, the metallic porous plate of the sample No. 12 was composed only of a porous body having a area ratio of holes of 65% in every surfaces vertical to the thickness direction and having a thickness of 1.5 mm), and was subjected to a tensile test.

As a result, the bonding strength of the metallic porous plate and the substrate was 60 MPa for the sample No. 7 and 35 MPa for the sample No. 12. The sample No. 7 in which the area ratio of the open holes (a) on the side facing the substrate was made less than the area ratio of the open holes (b) on the side facing the living body side had a value significantly higher than 22 MPa, which is a criterion determined by "Guidance for Industry on the Testing of Metallic Plasma Sprayed Coatings on Orthopedic Implants to Support Reconsideration of Postmarket Surveillance Requirements" of FDA (Food and Drug Administration). At the same time, the sample No. 7 retained sufficient bonding surface area between the metallic porous plate and the substrate as compared with the sample No. 12 in which the area ratio of the open holes on the side facing the substrate was the same as that of the open holes on the side facing the living body side, and thus had bonding strength sufficient for clinical use without any problem.

### INDUSTRIAL APPLICABILITY

The biocompatible metallic porous plate of the present invention is preferably used as a surface modification member for an implant material such as an artificial bone, an artificial joint, or an artificial dental root.

## Claims

1. A biocompatible metallic porous plate to be bonded to the surface of in vivo implant material, wherein
the area ratio A of open holes (a) present on the surface of the side facing the implant material is less than the area ratio B of open holes (b) present on the surface of the side facing a living body; and
the open holes (a) communicate through to the living body side.

2. The biocompatible metallic porous plate according to claim 1, which is produced by layered manufacturing.

3. The biocompatible metallic porous plate according to claim 1 or 2, wherein the area ratio of the open holes (a) present on the surface of the side facing the implant material is 0.4 to 30%, a distance between neighboring open holes (a) in the surface is 0.5 to 7.0 mm, and the open hole (a) has a diameter not exceeding 600 µm.

4. The biocompatible metallic porous plate according to any one of claims 1 to 3, wherein the open hole (a) has a thickness not exceeding 0.5 mm.

5. An in vivo implant material, wherein the biocompatible metallic porous plate according to any one of claims 1 to 4 is bonded to at least a portion of the surface of the in vivo implant material.

6. The in vivo implant material according to claim 5, wherein the biocompatible metallic porous plate is bonded by using a slurry containing metal powders.
